# EUROPEAN PATENT APPLICATION

(11) **EP 4 818 684 A1**
(43) Date of publication of application: **07.10.2026**
(21) Application number: 24897628.4
(22) Date of filing: 28.11.2024
(51) Int. Cl.: G01N 23/20008, C07C 63/28, C07D 213/75, G01N 23/207

(54) **MATERIAL FOR FORMING X-RAY STRUCTURE ANALYSIS SAMPLE, AND METHOD FOR DETERMINING MOLECULAR STRUCTURE OF ORGANIC COMPOUND USING SAME**

(30) Priority: 30.11.2023 JP 2023202754
(71) Applicant: The University of Tokyo, Bunkyo-ku, Tokyo 113-8654 (JP); Daicel Corporation, Osaka-shi, Osaka 530-0011 (JP)
(72) Inventor: SATO, Sota, Tokyo 113-8654 (JP); FUJITA, Makoto, Tokyo 113-8654 (JP); GONDO, Keisuke, Tokyo 108-8230 (JP)
(74) Representative: Grünecker Patent- und Rechtsanwälte PartG mbB
(86) International application number: PCT/JP2024/042092
(87) International publication number: WO 2025/115944

(57) **Abstract**

A novel material for producing an X-ray crystallographic sample for an organic compound is provided. The X-ray crystallographic sample-forming material of the present disclosure includes the following metal complex crystal. Metal complex crystal: The metal complex crystal being a metal complex crystal containing a metal ion and a ligand coordinated to the metal ion, the metal complex crystal having a three-dimensional network structure having regularly arranged cavities, the ligand containing a carboxylic acid-based ligand (c) and a pyridine-based ligand (p) in a combination of the following [I] or [II]. [I] A compound represented by the following Formula (cl) and a compound represented by the following Formula (pl). [II] A compound represented by the following Formula (c2) and a compound represented by the following Formula (p2).

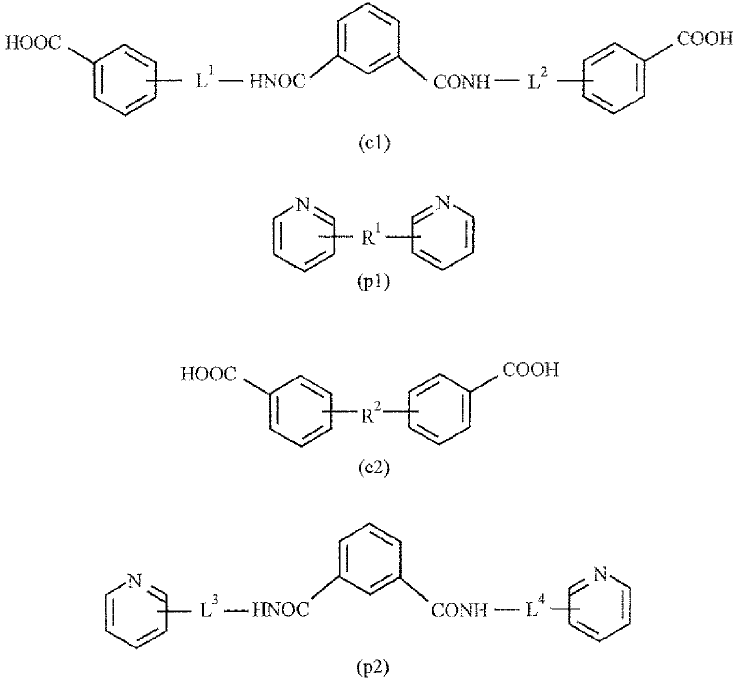

## Description

### Technical Field

The present disclosure relates to a novel X-ray crystallographic sample-forming material and a method for determining a molecular structure of an organic compound by using the X-ray crystallographic sample-forming material. The present disclosure claims priority from JP 2023-202754 filed in Japan on November 30, 2023, the contents of which are incorporated herein by reference.

### Background Art

As a method for determining a molecular structure of an organic compound, single crystal X-ray crystallography, that is, a method for subjecting a single crystal of the organic compound to X-ray crystallography, has been known. This method is significantly useful because the molecular structure of the organic compound can be accurately determined, if a high-quality single crystal can be prepared.

However, when the organic compound is in a very small amount, a sufficient amount of single crystal cannot be obtained, and determination of the molecular structure by this method is difficult. Furthermore, an organic compound having poor crystallizability exists, and this method cannot be employed for such an organic compound.

Thus, a crystalline sponge method has been developed that enables of determination of a molecular structure of an organic compound by X-ray crystallography without crystallizing the organic compound (e.g., Patent Document 1). The crystalline sponge method is a method for, by using a metal complex crystal having regularly arranged cavities as an X-ray crystallographic sample-forming material (or a crystalline sponge), impregnating the metal complex crystal with an organic compound whose molecular structure is desired to be determined and a solvent and fixing the organic compound in the cavities of the metal complex crystal, arranging the organic compound in a regularly arranged state to yield an X-ray crystallographic sample (i.e., a sample to be subjected to X-ray cystallography), irradiating the obtained X-ray crystallographic sample with X-rays, and thus, determining the molecular structure of the organic compound.

Furthermore, Non-Patent Literature 1 describes that a metal complex crystal having regularly arranged cavities can be obtained by reacting 1,3-bis(4-pyridylaminocarbonyl)benzene and 4,4'-dicarboxybiphenyl to zinc nitrate hydrate. However, use of the metal complex crystal as such an X-ray crystallographic sample-forming material has not been described.

### Citation List

### Patent Document

Patent Document 1: WO 2014/038220

### Non-Patent Literature

Non-Patent Literature 1: Optimization of Reaction Conditions towards Multiple Types of Framework Isomers and Periodic-Increased Porosity: Luminescence Properties and Selective CO2 Adsorption over N2, ChemPhysChem, 2013, 14, 3594-3599.

### Summary of Invention

### Technical Problem

When the metal complex crystal described in Patent Document 1 is used as an X-ray crystallographic sample-forming material, the rate of successful determination of the molecular structure of the organic compound is approximately 30%, and thus the low success rate is problematic.

Furthermore, although the success rate of the molecular structure determination can be increased by optimizing conditions, such as type of a solvent to be used during the impregnation of the organic compound into the metal complex crystal or the impregnation temperature, depending on the type of the organic compound, the metal complex crystal described in Patent Document 1 has low solvent resistance and dissolves in moderately polar to highly polar solvents, and thus had a problem in that the number of usable solvents is limited.

Furthermore, because inside of the cavities of the metal complex crystal described in Patent Document 1 is in a hydrophobic environment, it has been difficult to hold a hydrophilic organic compound in the cavities of the metal complex crystal, and it is difficult to determine the molecular structure of the hydrophilic organic compound using the metal complex crystal.

Furthermore, the metal complex crystal described in Patent Document 1 cannot maintain the crystal structure unless the cavities are filled with a solvent. Thus, in the metal complex crystal, a means could not have been adopted that the cavities are filled with a gas containing a gaseous organic compound and the gaseous organic compound is fixed within the cavities.

An object of the present disclosure is to provide a novel material for forming an X-ray crystallographic sample for an organic compound. The material can regularly arrange and fix the organic compound.

Another object of the present disclosure is to provide a novel material for forming an X-ray crystallographic sample for an organic compound, the material enabling an hydrophilic organic compound to be regularly arranged and fixed.

Another object of the present disclosure is to provide a novel material for forming an X-ray crystallographic sample for an organic compound, the material enabling a gaseous organic compound present in the air to be regularly arranged and fixed.

Another object of the present disclosure is to provide a method for determining a molecular structure of an organic compound, the method using the material.

Another object of the present disclosure is to provide a method for determining a molecular structure of an odor component, the method using the material.

### Solution to Problem

As a result of diligent research to solve the problems described above, the present inventors have discovered the following.

To determine a molecular structure of an organic compound, the organic compound needs to be regularly arranged; and however, in the metal complex crystal described in Patent Document 1, the organic compound is fixed in the cavities by relatively weak intermolecular interaction, such as Π-Π interaction or σ-Π interaction, and thus the probability of holding the organic compound in the cavities is low. It was found that the metal complex crystal described in Patent Document 1 has a weak function of regularly arranging an organic compound, and thus, when the metal complex crystal is used as a crystalline sponge, the probability of successful determination of the molecular structure is low.

Meanwhile, it was found that an amide group can strongly interact with various functional groups due to a hydrogen bond; when a particular ligand having an amide group is coordinated with a metal ion, a metal complex crystal having regularly arranged cavities can be obtained; the regularly arranged cavities of the metal complex crystal thus obtained can house the organic compound with a high probability and the housed organic compound is firmly held in an oriented state, and thus, a molecular structure of an organic compound can be determined in high probability by an X-ray diffraction method when the metal complex crystal is used as an X-ray crystallographic sample-forming material. The present disclosure was completed based on these findings.

That is, the present disclosure provides a material for forming an X-ray crystallographic sample, the material being an X-ray crystallographic sample-forming material containing an organic compound in a regularly arranged state, and the material containing a metal complex crystal described below:
Metal complex crystal: The metal complex crystal being a metal complex crystal containing a metal ion and a ligand coordinated to the metal ion, the metal complex crystal having a three-dimensional network structure having regularly arranged cavities, the ligand containing a carboxylic acid-based ligand (c) and a pyridine-based ligand (p), and the carboxylic acid-based ligand (c) and the pyridine-based ligand (p) being in a combination of the following [I] or [II]:
[I] A compound represented by the following Formula (c1) and a compound represented by the following Formula (p1); or
[II] A compound represented by the following Formula (c2) and a compound represented by the following Formula (p2): 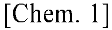 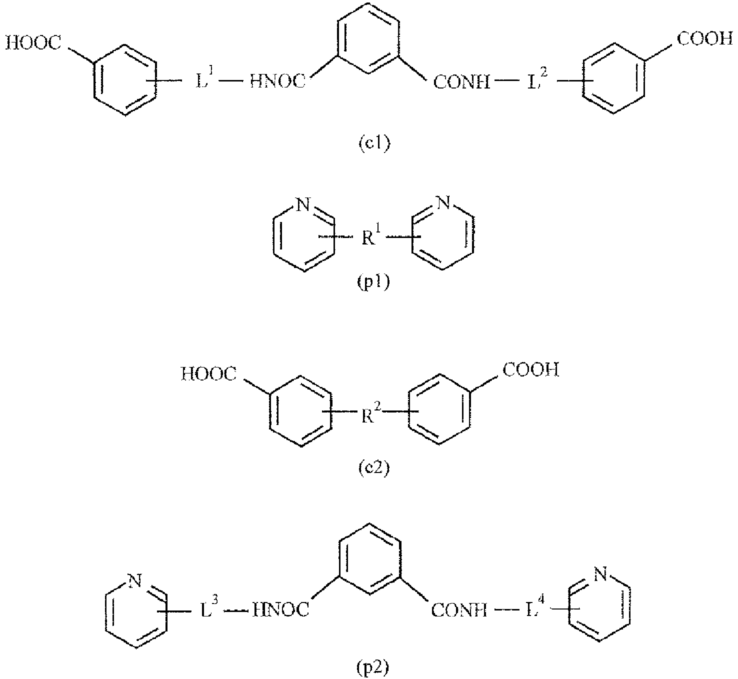 where R¹ and R² are the same or different and each represent a group selected from a single bond, a divalent hydrocarbon group, a divalent heterocyclic group, or a divalent group containing two or more of the groups, the two or more of the groups being linked. L¹ to L⁴ are the same or different and each represent a single bond or a linking group.

The present disclosure also provides the X-ray crystallographic sample-forming material, wherein the metal ion is a metal ion selected from a zinc ion, an iron ion, a cobalt ion, a nickel ion, a copper ion, or a silver ion.

The present disclosure also provides the X-ray crystallographic sample-forming material, wherein the organic compound is an organic compound including a functional group capable of interacting with at least one group selected from a CONH group, a CO group, and an NH group.

The present disclosure also provides the X-ray crystallographic sample-forming material, wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.

The present disclosure also provides the X-ray crystallographic sample-forming material, wherein the organic compound is an organic compound having a log P value of -5 or greater and 7 or less.

The present disclosure also provides a method for determining a molecular structure of an organic compound (X), the method including the following steps 1 and 2:
step 1: allowing the organic compound (X) to infiltrate into the X-ray crystallographic sample-forming material, and fixing the organic compound (X) in cavities of the X-ray crystallographic sample-forming material; and
step 2: irradiating the X-ray crystallographic sample-forming material containing the fixed organic compound (X) with X-rays, analyzing obtained diffraction data, and determining a molecular structure of the organic compound (X).

The present disclosure also provides a method for determining a molecular structure of a gaseous odor component (Y), the method including the following steps 1, 2, and 3:
step 1: feeding a sample gas containing the gaseous odor component (Y) to a column for separating gas components and fractionating the gaseous odor component (Y);
step 2: allowing the fractionated gaseous odor component (Y) to infiltrate into the X-ray crystallographic sample-forming material, and fixing the gaseous odor component (Y) in cavities of the X-ray crystallographic sample-forming material; and
step 3: irradiating the X-ray crystallographic sample-forming material containing the fixed gaseous odor component (Y) with X-rays, analyzing obtained diffraction data, and determining a molecular structure of the gaseous odor component (Y).

### Advantageous Effects of Invention

The X-ray crystallographic sample-forming material of the present disclosure includes a metal complex crystal having large, regularly arranged cavities.

The cavities of the metal complex crystal are not fixed in shape and have deformability such that the cavities deform depending on the shape of an organic compound to be incorporated (so-called guest molecule). Thus, various organic compounds can be incorporated into the cavities of the metal complex crystal with a high probability.

Furthermore, the metal complex crystal includes an amide group, and the amide group is a group having a property of forming hydrogen bonds with various functional groups. Thus, the organic compound incorporated into the cavities of the metal complex crystal can be firmly fixed with a high probability in a regularly arranged state.

Furthermore, because the metal complex crystal has an amide group, cavities in the metal complex crystal exhibit hydrophilic environment, and a hydrophilic organic compound can be incorporated into a cavities with a high probability and fixed.

Furthermore, the metal complex crystal has excellent solvent resistance, and can maintain the crystal structure in various solvents.

Furthermore, the metal complex crystal has excellent toughness and can maintain the crystal structure regardless of whether the cavities are filled with a solvent or not filled with a solvent (i.e., filled with air).

Thus, the cavities of the X-ray crystallographic sample-forming material can not only allow for infiltration of a liquid organic compound (X) (or infiltration of a solid organic compound (X) dissolved in a solvent) and fixing of the liquid organic compound (X) in the cavities, but also infiltrate a gaseous organic compound (X) and fix the gaseous organic compound (X) in the cavities.

Thus, when the X-ray crystallographic sample-forming material is used, the organic compound (X) in a solid, liquid, or gaseous state can be fixed in the cavities with a high probability, and the organic compound (X) can be regularly arranged in the metal complex crystal.

When the X-ray crystallographic sample-forming material is used, even in the case where the amount of the organic compound (X) is too small to form a single crystal or even in the case of a compound that cannot form a single crystal, the organic compound (X) can be regularly arranged in the cavities of the X-ray crystallographic sample-forming material, and the molecular structure of the organic compound (X) can be determined by subjecting the X-ray crystallographic sample thus obtained to X-ray crystallography.

Thus, the X-ray crystallographic sample-forming material is extremely useful as a means for determining a molecular structure of a solid, liquid, or gaseous organic compound (X) (especially, hydrophilic organic compound) by X-ray crystallography.

### Brief Description of Drawings

FIG. 1 is a schematic view illustrating a crystal structure of a sample-forming material 1 obtained in Example.
FIG. 2 is a schematic view illustrating a crystal structure of a sample-forming material 4 obtained in Comparative Example.
FIG. 3(a) is a schematic view of a crystal structure of an X-ray crystallographic sample (metal complex crystal in which benzil is fixed in the cavities) obtained in Example 14. FIG. 3(b) is a schematic view of a shape of a cavity of the X-ray crystallographic sample. FIG. 3(c) illustrates interaction between N-H comprised in an amide group of the sample-forming material 1 and an oxygen atom of an oxo group contained in the benzil.
FIG. 4(a) is a schematic view of a crystal structure of an X-ray crystallographic sample (metal complex crystal in which dibutyl fumarate is fixed in the cavities) obtained in Example 22. FIG. 4(b) is a schematic view of a shape of cavity of the X-ray crystallographic sample. FIG. 4(c) illustrates interaction between N-H comprised in an amide group of the sample-forming material 1 and an oxygen atom of an oxo group contained in the dibutyl fumarate.
FIG. 5 is a schematic view illustrating analysis equipment used in determination of a structure of an odor component of Example. For the analysis equipment, a branching tool is equipped immediately before a detector of a gas chromatograph and is connected to fractionating device.

### Description of Embodiments

### X-ray Crystallographic Sample-Forming Material

The X-ray crystallographic sample-forming material of the present disclosure (hereinafter, may be referred to as "sample-forming material") may be referred to as a crystalline sponge, is a material for forming a sample to be subjected to X-ray crystallography, and is a material used for fixing an organic compound in a regularly arranged state.

The sample-forming material at least contains the following metal complex crystal. The sample-forming material may contain an additional component; and however, the proportion of the metal complex crystal described below is, for example, 50 wt.% or greater, preferably 60 wt.% or greater, more preferably 70 wt.% or greater, even more preferably 80 wt.% or greater, particularly preferably 90 wt.% or greater, most preferably 95 wt.% or greater, and especially preferably 99 wt.% or greater, with respect to the total amount of the sample-forming material. The sample-forming material may contain substantially only the following metal complex crystal.

### Metal Complex Crystal

The metal complex crystal comprises a metal ion and a ligand coordinated to the metal ion and is a metal complex crystal having a three-dimensional network structure including regularly arranged cavities (preferably microporous metal complex crystal).

The metal ion is not particularly limited as long as such a three-dimensional network structure can be formed by a coordinate bond of a ligand described below. Examples of the metal ion include ions of metals of Groups 8 to 12 in the periodic table, such as a zinc ion, an iron ion, a cobalt ion, a nickel ion, a copper ion, and a silver ion. In particular, a divalent metal ion is preferred, and a zinc ion and/or a cobalt ion is particularly preferred, in that a metal complex crystal having a three-dimensional network structure having large cavities is obtained.

The ligand contains at least one carboxylic acid-based ligand (c) and at least one pyridine-based ligand (p).

The combination of the carboxylic acid-based ligand (c) and the pyridine-based ligand (p) is the following [I] or [II].
[I] A compound represented by the following Formula (c1) and a compound represented by the following Formula (p1)
[II] A compound represented by the following Formula (c2) and a compound represented by the following Formula (p2) 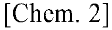 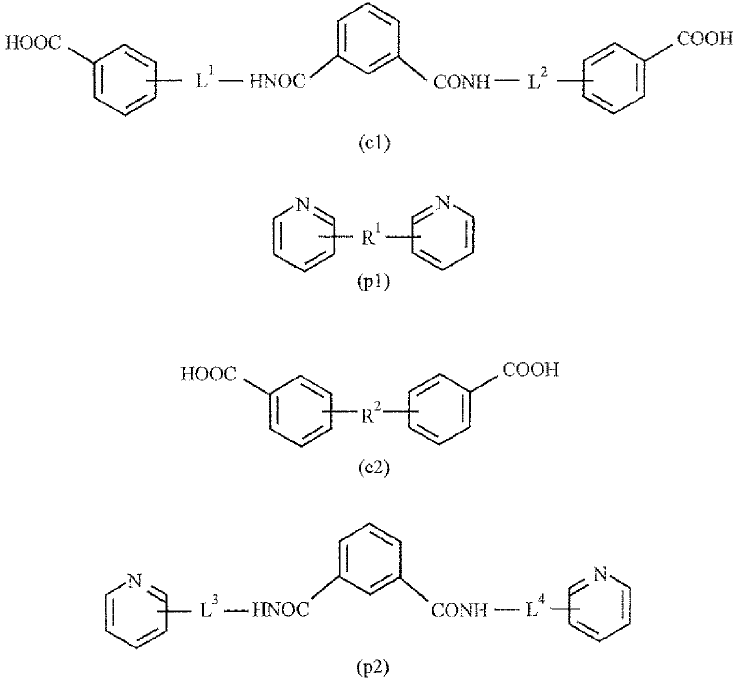
where R¹ and R² are the same or different and each represent a group selected from a single bond, a divalent hydrocarbon group, a divalent heterocyclic group, or a divalent group containing two or more of the groups, the two or more of the groups being linked. L¹ to L⁴ are the same or different and each represent a single bond or a linking group.

Examples of the divalent hydrocarbon group of R¹ and R² include linear or branched alkylene groups having from 1 to 18 carbons, such as a methylene group, a methylmethylene group, a dimethylmethylene group, an ethylene group, a propylene group, and a trimethylene group; cycloalkylene groups having from 3 to 18 carbons, such as a 1,2-cyclopentylene group, a 1,3-cyclopentylene group, a cyclopentylidene group, a 1,2-cyclohexylene group, a 1,3-cyclohexylene group, a 1,4-cyclohexylene group, and a cyclohexylidene group; and C₆₋₁₄ arylene groups, such as a phenylene group (e.g., an o-phenylene group, an m-phenylene group, and a p-phenylene group), a phenylenebis(methylene) group (e.g., a 1,2-phenylenebis(methylene) group, a 1,3-phenylenebis(methylene group), and a 1,4-phenylenebis(methylene group)), a biphenylene group, a naphthylene group, a binaphthylene group, an anthracenylene group, and a phenanthrenylene group.

The divalent heterocyclic group of R¹ and R² has a structural formula of a heterocyclic ring with two hydrogen atoms removed. Examples of the heterocyclic ring include an aromatic heterocyclic ring and a non-aromatic heterocyclic ring. Examples of the heterocyclic ring include 3 to 20-membered rings (preferably 3 to 10-membered rings and particularly preferably 4 to 6-membered rings) having carbon atoms and at least one heteroatom (e.g., an oxygen atom, a sulfur atom, and a nitrogen atom) as ring atoms, and condensed rings thereof.

Examples of the heterocyclic ring containing an oxygen atom as the heteroatom include 3-membered rings, such as an oxirane ring; 4-membered rings, such as an oxetane ring; 5-membered rings, such as a furan ring, a tetrahydrofuran ring, an oxazole ring, an isoxazole ring, and a γ-butyrolactone ring; 6-membered rings, such as a 4-oxo-4H-pyran ring, a tetrahydropyran ring, and a morpholine ring; condensed rings, such as a benzofuran ring, an isobenzofuran ring, a 4-oxo-4H-chromene ring, a chroman ring, and an isochroman ring; bridged rings, such as a 3-oxatricyclo[4.3.1.1^{4,8}]undecan-2-one ring and a 3-oxatricyclo[4.2.1.0^{4,8}]nonan-2-one ring.

Examples of the heterocyclic ring containing a sulfur atom as the heteroatom include 5-membered rings, such as a thiophene ring, a thiazole ring, an isothiazole ring, and a thiadiazole ring; 6-membered rings, such as a 4-oxo-4H-thiopyran ring; and condensed rings, such as a benzothiophene ring.

Examples of the heterocyclic ring containing a nitrogen atom as the heteroatom include 5-membered rings, such as a pyrrole ring, a pyrrolidine ring, a pyrazole ring, an imidazole ring, and a triazole ring; 6-membered rings, such as an isocyanuric ring, a pyridine ring, a pyridazine ring, a pyrimidine ring, a pyrazine ring, a piperidine ring, and a piperazine ring; and condensed rings, such as an indole ring, an indoline ring, a quinoline ring, an acridine ring, a naphthyridine ring, a quinazoline ring, and a purine ring.

L¹ to L⁴ are the same or different and each represent a single bond or a linking group. The linking group is a divalent group having one or more atoms, and examples thereof include a divalent hydrocarbon group.

Examples of the divalent hydrocarbon group include linear or branched alkylene groups having from 1 to 18 carbons, such as a methylene group, a methylmethylene group, a dimethylmethylene group, an ethylene group, a propylene group, and a trimethylene group; cycloalkylene groups having from 3 to 18 carbons, such as a 1,2-cyclopentylene group, a 1,3-cyclopentylene group, a cyclopentylidene group, a 1,2-cyclohexylene group, a 1,3-cyclohexylene group, a 1,4-cyclohexylene group, and a cyclohexylidene group; C₆₋₁₄ arylene groups, such as a phenylene group (e.g., an o-phenylene group, an m-phenylene group, and a p-phenylene group), a naphthylene group (e.g., a 1,3-naphthylene group, a 1,4-naphthylene group, and a 2,7-naphthylene group), an anthracenylene group, and a phenanthrenylene group; and a group in which two or more thereof are linked [e.g., a phenylenebis(methylene) group (e.g., a 1,2-phenylenebis(methylene) group, a 1,3-phenylenebis(methylene) group, and a 1,4-phenylenebis(methylene) group), a biphenylene group, and a binaphthylene group].

The divalent hydrocarbon group is preferably a C₆₋₁₄ arylene group or a group in which two or more of the arylene groups are bonded through a single bond or a C₁₋₅ alkylene group.

The benzene ring and the pyridine ring in Formulas (c1), (p1), (c2), and (p2) may have a substituent besides the groups represented in the formulas.

Examples of the substituent include halogen atoms, oxo groups, hydroxyl groups, substituted oxy groups (e.g., C₁₋₄ alkoxy groups), cyano groups, nitro groups, substituted or unsubstituted amino groups (e.g., mono- or di-C₁₋₄ alkylamino groups, such as a methylamino group, a dimethylamino group, an ethylamino group, and a diethylamino group; 5 to 8-membered cyclic amino groups, such as a 1-pyrrolidinyl group, a piperidino group, and a morpholino group; C₁₋₁₀ acylamino groups, such as an acetylamino group, a propionylamino group, and a benzoylamino group; and sulfonylamino groups, such as a benzenesulfonylamino group and a p-toluenesulfonylamino group), sulfonic acid groups, alkyl groups (e.g., C₁₋₄ alkyl groups, such as a methyl group and an ethyl group), alkenyl groups (e.g., C₂₋₄ alkenyl groups, such as a vinyl group, an allyl group, and a 1-butenyl group), alkynyl groups (e.g., C₂₋₄ alkynyl groups, such as an ethynyl group and a propynyl group), C₃₋₈ cycloalkyl groups, and aryl groups (e.g., C₆₋₁₀ aryl groups, such as a phenyl group and a naphthyl group).

When the benzene ring or the pyridine ring in Formulas (c1), (p1), (c2), and (p2) has two or more substituents, the two groups selected from the substituents described above may be bonded each other to form a ring.

Furthermore, an aromatic or non-aromatic hydrocarbon ring or heterocyclic ring may be fused to the benzene ring or the pyridine ring in Formulas (c1), (p1), (c2), and (p2).

Specific examples of the compound represented by Formula (c1) above include a compound represented by the following Formula (c1-1), a compound represented by Formula (c1-2), a compound represented by Formula (c1-3), and a compound represented by Formula (c1-4) as described below. Examples of the compound represented by Formula (c1-1) below include a compound having CONH groups corresponding to the CONH-L¹ group and the CONH-L² group in Formula (c1), the CONH groups being bonded at the ortho-position, the meta-position, or the para-position with respect to the bonding positions of the carboxyl groups in the benzene rings. The same applies to the compounds represented by the following Formulas (c1-2) to (c1-4).

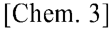

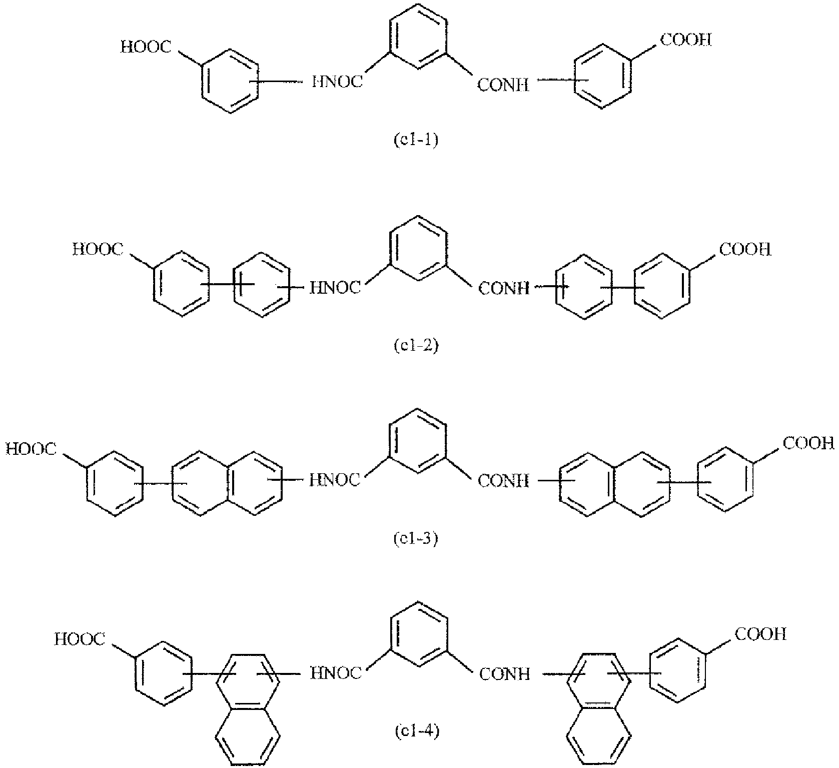

As the compound represented by Formula (c1), the compound represented by Formula (c1-1) is preferred in that a metal complex crystal having a three-dimensional network structure with large cavities is obtained.

Specific examples of the compound represented by Formula (p1) include: compounds having a group corresponding to the R¹ group in Formula (p1), the group being bonded at the para-position with respect to the nitrogen atoms of the pyridine rings, such as a compound represented by Formula (p1-1), a compound represented by Formula (p1-2), a compound represented by Formula (p1-3), a compound represented by Formula (p1-4), a compound represented by Formula (p1-5), a compound represented by Formula (p1-6), and a compound represented by Formula (p1-7) as described below; and compounds corresponding to the aforementioned compounds and having a group corresponding to the R¹ group in Formula (p1), the group being bonded to the ortho-position or the meta-position with respect to the nitrogen atoms of the pyridine rings.

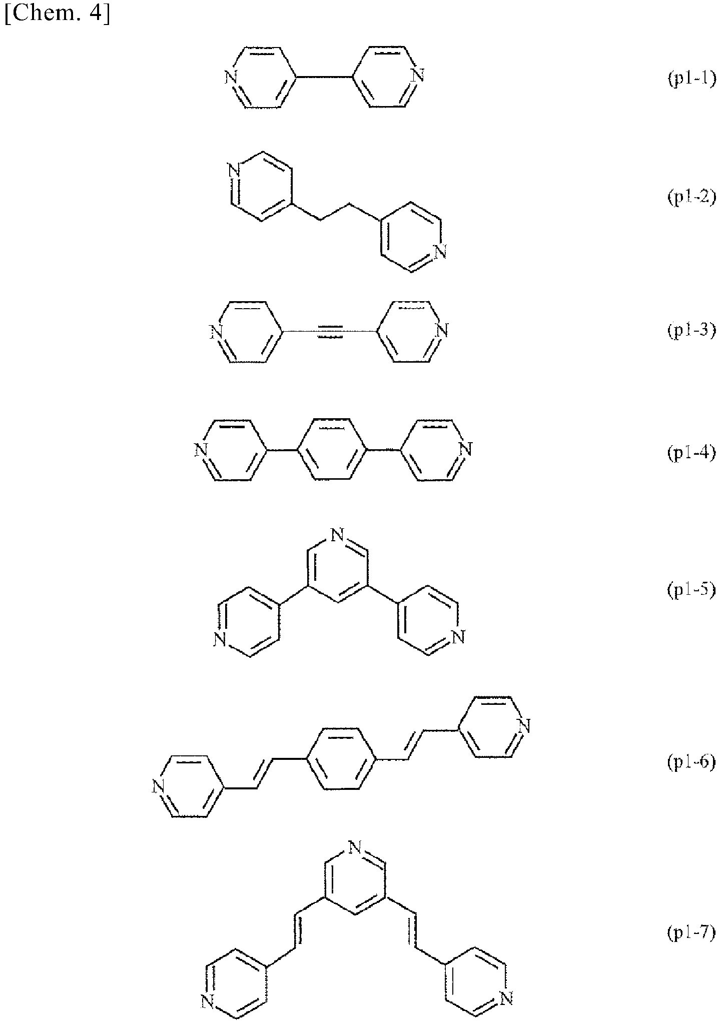

As the compound represented by Formula (p1), the compound represented by Formula (p1-1), the compound represented by Formula (p1-2), the compound represented by Formula (p1-5), and the compound represented by Formula (p1-6) are preferred, and the compound represented by Formula (p1-1) and the compound represented by Formula (p1-2) are particularly preferred, in that a metal complex crystal having a three-dimensional network structure with large cavities is obtained.

Specific examples of the compound represented by Formula (c2) include a compound represented by the following Formula (c2-1), a compound represented by Formula (c2-2), a compound represented by Formula (c2-3), a compound represented by Formula (c2-4), and a compound represented by Formula (c2-5) as described below. Examples of the compound represented by Formula (c2-1) below include a compound having a carboxyl group bonded at the ortho-position, the meta-position, or the para-position with respect to the linking part of the benzene rings. The same applies to the compounds represented by the following Formulas (c2-2) to (c2-5).

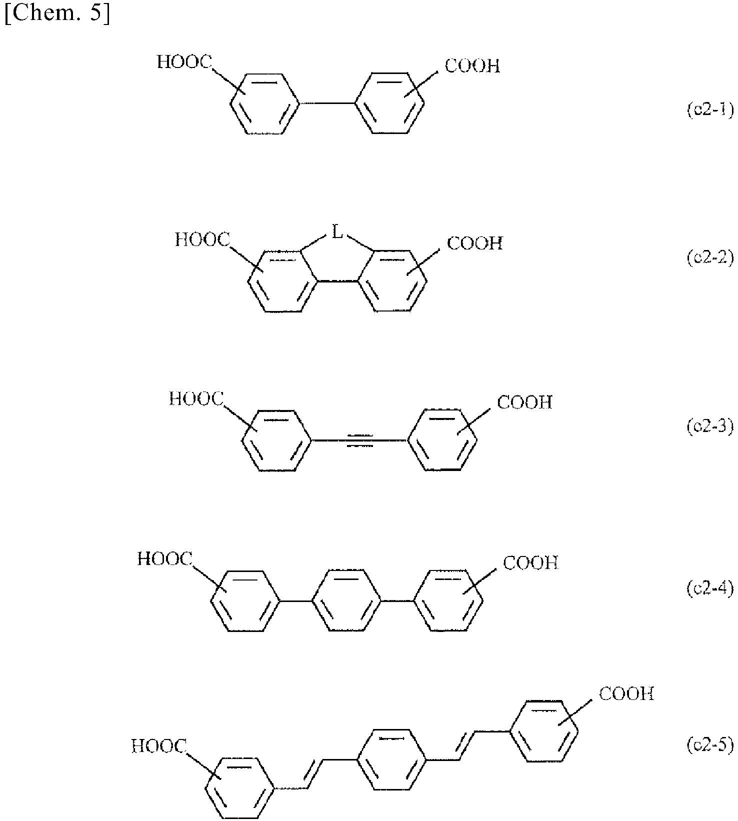

In Formula (c2-2) above, L represents a linking group. The linking group is a divalent group having at least one atom, and examples thereof include a carbonyl group (-CO-), an ether bond (-O-), a thioether bond (-S-), an ester bond (-COO-), an amide bond (-CONH-), an imino group (-NH-), and a sulfonyl group (-S(=O)₂-).

As the compound represented by Formula (c2), the compound represented by Formula (c2-1) and the compound represented by Formula (c2-2) are preferred, and the compound represented by Formula (c2-1) is particularly preferred, in that a metal complex crystal having a three-dimensional network structure with large cavities is obtained.

Specific examples of the compound represented by Formula (p2) include a compound represented by Formula (p2-1), a compound represented by Formula (p2-2), a compound represented by Formula (p2-3), and a compound represented by Formula (p2-4). Examples of the compound represented by Formula (p2-1) below include a compound having groups corresponding to the CONH-L³ group and the CONH-L⁴ group in Formula (p2), the groups being bonded at the ortho-position, the meta-position, or the para-position with respect to the nitrogen atoms in the pyridine rings. The same applies to the compounds represented by the following Formulas (p2-2) to (p2-4).

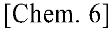

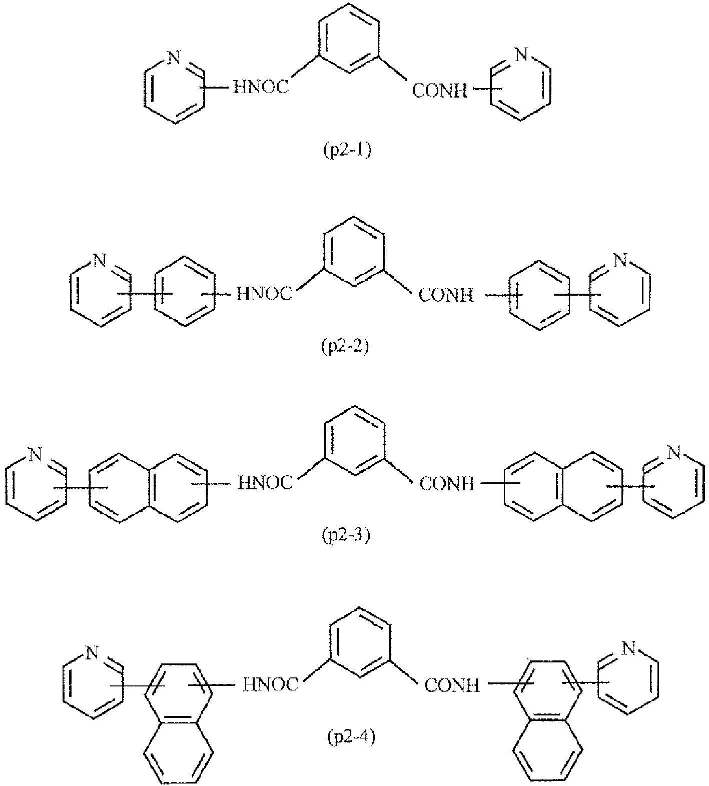

The compound represented by Formula (p2) is preferably a compound represented by Formula (p2-1), in that a metal complex crystal having a three-dimensional network structure with large cavities is obtained.

The ligand comprised in the metal complex crystal contains the carboxylic acid-based ligand (c) and the pyridine-based ligand (p), and the content ratio of the carboxylic acid-based ligand (c) to the pyridine-based ligand (p) [carboxylic acid-based ligand (c)/pyridine-based ligand (p); molar ratio] is, for example, from 30/70 to 70/30, preferably from 40/60 to 60/40, and particularly preferably from 45/55 to 55/45, in that a metal complex crystal having a three-dimensional network structure with large cavities is obtained.

The content ratio of the metal ion to the carboxylic acid-based ligand (c) [metal ion/carboxylic acid-based ligand (c); molar ratio] is, for example, from 30/70 to 70/30, preferably from 40/60 to 60/40, and particularly preferably from 45/55 to 55/45.

The content ratio of the metal ion to the pyridine-based ligand (p) [metal ion/pyridine-based ligand (p); molar ratio] is, for example, from 30/70 to 70/30, preferably from 40/60 to 60/40, and particularly preferably from 45/55 to 55/45.

The ligand may contain an additional ligand besides the carboxylic acid-based ligand (c) and the pyridine-based ligand (p). Examples of the additional ligand include anions such as F⁻, Cl⁻, Br⁻, I⁻, SCN⁻, NO₃⁻, ClO₄⁻, BF₄⁻, SbF₄⁻, PF₆⁻, AsF₆⁻, and CH₃COO⁻.

As the anions, anions selected from NO₃⁻, F⁻, Cl⁻, Br⁻, or I⁻ are preferred, and NO₃⁻ is particularly preferred, in that a metal complex crystal having a three-dimensional network structure with large cavities is obtained.

The proportion of the total content of the carboxylic acid-based ligand (c) and the pyridine-based ligand (p) with respect to the total amount of the ligands comprised in the metal complex crystal is, for example, 50 wt.% or greater, preferably 60 wt.% or greater, more preferably 70 wt.% or greater, even more preferably 80 wt.% or greater, and particularly preferably 90 wt.% or greater.

The metal complex crystal has a three-dimensional network structure having regularly arranged cavities. Each of the cavities is a space present inside the metal complex crystal and is a space partitioned by a network structure made of metal ions and ligands.

The cavities are preferably regularly arranged three-dimensionally without irregularity to the degree that can be confirmed by X-ray crystallography.

The shape of each of the cavities is not particularly limited; and however, each of the cavities preferably has a fixed shape and a fixed size to the degree that can be confirmed by X-ray crystallography.

The shape of each of the cavities has deformability such that each of the cavities deforms depending on the shape of the organic compound (X) to be incorporated into each of the cavities. This is because the metal complex crystal is not formed by a series of three-dimensional network structures but is formed by a plurality (e.g., 3 or more, and preferably 4 or more) of independent three-dimensional network structures interpenetrating each other.

The size of each of the cavities present in single unit cell preferably has a volume that can contain one to some moieties (e.g., from 1 to 6) of organic compounds (X).

The size of a cavity correlates with a diameter of an inscribed circle of the cavity on a plane parallel to the crystal plane that is as close as possible to being perpendicular to the extending direction of the cavity (hereinafter, such a plane may be referred to as "parallel plane"). Hereinafter, such an inscribed circle may be simply referred to as an "inscribed circle of the cavity. When the inscribed circle is larger, the cavity is larger. When the inscribed circle is smaller, the cavity is smaller.

The "extending direction of the cavity" can be determined by the following method.

First, a crystal plane 1 (one of six crystal planes connecting the four lattice points included in one unit cell) in a proper direction that intersects a target cavity is selected. A cross-sectional view of the cavity with the crystal face 1 as a cutting plane is drawn by representing an atom that is comprised in the metal complex crystal and is present on the crystal plane 1, using a van der Waals radius. Similarly, a cross-sectional view of the cavity is drawn using, as a cutting plane, a crystal plane 2 that is shifted by one unit cell from the crystal plane 1. Then, the centers of the cross-sectional shapes of the cavities of the respective crystal planes are connected by a straight line in a three-dimensional view. The direction of the straight line obtained at this time is the extending direction of the cavity.

Furthermore, the "diameter of the inscribed circle of the cavity" can be determined by the following method.

First, a cross-sectional view of the cavity is drawn by the method described above. Next, an inscribed circle is drawn in the cross-sectional view, and after the diameter of the inscribed circle is measured, the obtained measured value is converted into the actual scale, and thus, the actual diameter of the inscribed circle of the cavity can be determined.

Furthermore, while the parallel face is gradually moved parallel within one unit cell, cross-sectional views of the cavity in the respective parallel faces are drawn, and by measuring the diameters of the inscribed circles, the diameter of the inscribed circle at the narrowest part and the diameter of the inscribed circle of the widest part can be determined.

The diameter of the inscribed circle of the cavities is, for example, from 8 to 12 Å, and preferably from 9 to 12 Å.

Furthermore, when the cavities have a shape significantly different from a perfect circle, the size of the respective cavities is preferably estimated based on the minor axis and the major axis of an inscribed ellipse of each of the cavities in the aforementioned parallel face.

The major axis of the inscribed ellipse of each of the cavities is preferably from 10 to 15 Å, and more preferably from 10 to 12 Å. Furthermore, the minor axis of the inscribed ellipse of each of the cavities is preferably from 8 to 15 Å, and more preferably from 8 to 11 Å.

Although the metal complex crystal contains the ligand containing an amide group (CONH) as a constituent component, the amide group (CONH group) and the CO group and the NH group comprised in the amide group can interact with various functional groups through a hydrogen bond. Thus, the metal complex crystal can determine molecular structures of a wide range of organic compounds or can determine molecular structures of more kinds of organic compounds. The metal complex crystal can firmly fix the organic compound in the cavities by a hydrogen bond with the amide group, the CO group, or the NH group. Thus, when the metal complex crystal is used, molecular structures of various organic compounds having functional groups that can interact with the amide group, the CO group, or the NH group can be determined with a high probability.

Furthermore, the metal complex crystal has excellent durability, and the crystal structure can be maintained not only in an solution, but also in an air atmosphere. Thus, even in an organic compound having volatility and being gasified and dispersed in a gas phase, the metal complex crystal can incorporate such an organic compound into the cavities of the metal complex crystal and can fix the organic compound. Accordingly, the metal complex crystal can be used for determination of a molecular structure by subjecting an odor component in a gas state to infiltration and fixing the odor component, and thus has excellent versatility.

### Method for Producing X-ray Crystallographic Sample-Forming Material

The X-ray crystallographic sample-forming material can be produced by using the following method for producing a metal complex crystal.

### Method for Producing Metal Complex Crystal

The metal complex crystal can be produced by reacting a carboxylic acid-based ligand (c), a pyridine-based ligand (p), and a salt of a metal ion and a counter ion (hereinafter, may be referred to as "metal salt") in the presence of a solvent.

Examples of the counter ion include anions such as F⁻, Cl⁻, Br⁻, I⁻, SCN⁻, NO₃⁻, ClO₄⁻, BF₄⁻, SbF₄⁻, PF₆⁻, AsF₆⁻, and CH₃COO⁻.

The used ratio of the carboxylic acid-based ligand (c) to the pyridine-based ligand (p) [carboxylic acid-based ligand (c)/pyridine-based ligand (p); molar ratio] is, for example, from 30/70 to 70/30. The used ratio is preferably from 40/60 to 60/40, and particularly preferably from 45/55 to 55/45,, in that a metal complex crystal having a three-dimensional network structure with large cavities is obtained.

The used amount of the metal salt with respect to 1 mol of the carboxylic acid-based ligand (c) or the pyridine-based ligand (p) is, for example, from 0.2 to 3 mol, preferably from 0.5 to 2 mol, and particularly preferably from 0.8 to 1.5 mol.

Examples of the solvent include water; benzene; benzene derivatives replaced at least one hydrogen atom bonded to a benzene ring with, for example, a halogen atom, an alkyl group, a haloalkyl group, an alkoxy group, a cyano group, a nitro group, or a substituted oxycarbonyl group, such as toluene, xylene, ethylbenzene, trifluoromethylbenzene (or trifluorotoluene), chlorobenzene, anisole, benzonitrile, nitrobenzene, and ethyl benzoate; aliphatic hydrocarbons, such as hexane, heptane, and octane; alicyclic hydrocarbons, such as cyclohexane; haloalkane, such as carbon tetrachloride, chloroform, dichloromethane, and 1,2-dichloroethane; ketones, such as acetone, methyl ethyl ketone; esters, such as methyl acetate, ethyl acetate, isopropyl acetate, and butyl acetate; amides, such as N,N-dimethylformamide (DMF), N,N-dimethylacetamide, and N-methylpyrrolidone; nitriles, such as acetonitrile and propionitrile; acyclic or cyclic ethers, such as diethyl ether, dibutyl ether, dimethoxyethane, dioxane, and tetrahydrofuran; and organic acids, such as acetic acid. One type of these may be used alone, or two or more types thereof may be used in combination.

In particular, the solvent is preferably a liquid mixture of water and an amide (especially, DMF). The volume ratio of the water with respect to the total volume of the water and the amide is, for example, from 5 to 95 vol%. The lower limit value of the volume ratio is preferably 10 vol%, and particularly preferably 15 vol%, and the upper limit value of the volume ratio is preferably 80 vol%, particularly preferably 60 vol%, more preferably 50 vol%, even more preferably 40 vol%, particularly preferably 30 vol%, most preferably 25 vol%, and especially preferably 17 vol%, in that a metal complex crystal having a three-dimensional network structure with large cavities is obtained.

The reaction temperature is, for example, from 60 to 150°C, preferably from 80 to 120°C, and particularly preferably from 90 to 110°C.

The reaction time is, for example, from 0.5 to 10 hours, preferably from 1 to 8 hours, particularly preferably from 2 to 6 hours, and most preferably from 3 to 5 hours.

The reaction atmosphere is not particularly limited as long as the reaction is not inhibited, and may be, for example, any of an air atmosphere, a nitrogen atmosphere, and an argon atmosphere.

After completion of the reaction, the obtained reaction product may be subjected to separation and purification by ordinary precipitation, washing, and filtration.

### Method for Determining Molecular Structure of Organic Compound (X)

The method for determining the molecular structure of the organic compound (X) includes the following step 1 and step 2.

Step 1: allowing the organic compound (X) to infiltrate into the sample-forming material (or the metal complex crystal), and fixing the organic compound (X) in cavities of the sample-forming material (or the metal complex crystal).

Step 2: irradiating the sample-forming material (or the metal complex crystal) containing the fixed organic compound (X) with X-rays, analyzing obtained diffraction data, and determining a molecular structure of the organic compound (X).

The organic compound (X) is, for example, a compound having at least one functional group capable of interacting with an amide group (CONH group) contained in the sample-forming material (or the metal complex crystal) or a CO group or an NH group comprised in the amide group, through a hydrogen bond.

Examples of the functional group capable of interacting with at least one group selected from the CONH group, the CO group, and the NH group include a group having electronegativity of 1.0 to 4.0 (preferably 1.6 to 3.6, and particularly preferably 2.0 to 3.4).

Examples of the functional group include a substituted or unsubstituted amino group, a carboxyl group, an active methylene group, a nitrile group, a ketone group, an aldehyde group, an ester group, an ether group, an amide group, a hydroxy group, a halogen group, and a sulfonamide group.

The molecular weight of the organic compound (X) is, for example, 1000 or less, preferably 800 or less, and particularly preferably 600 or less. The lower limit value of the molecular weight of the organic compound (X) is, for example, 15, preferably 30, and particularly preferably 50.

The organic compound (X) is preferably a hydrophilic compound, and the log P (octanol/water partition coefficient) value of the organic compound (X) is, for example, -5 or greater and 7 or less. From the viewpoint of improving a rate of successful determination of a molecular structure, the lower limit value of the log P is preferably -3, and particularly preferably -1, and the upper limit value of the log P is preferably 6, particularly preferably 5, most preferably 4.5, and especially preferably 3.

Note that the log P value of the organic compound (X) can be determined by using a commercially available software (e.g., "EPI suite", the software jointly developed by United States Environmental Protection Agency (US EPA) (The Estimations Programs Interface for Windows) and SRC, Inc.).

### (Step 1)

The step 1 is a step of allowing the organic compound (X) to infiltrate into the sample-forming material, and fixing the organic compound (X) in cavities of the sample-forming material.

From the viewpoint of improving rate of successful determination of a molecular structure, as the sample-forming material, a sample having excellent transparency and a clear external shape (examples of the shape include a needle-like shape, a block-like shape, and a flake-like shape) is preferably selected and used among the metal complex crystals obtained by the method for producing the metal complex crystal described above.

In the case where the sample-forming material has a needle-like or block-like shape, the length of the sample-forming material is, for example, 10 µm or greater, preferably from 10 to 200 µm, and particularly preferably from 10 to 100 µm. When the sample-forming material is too small, the measurement time tends to be longer. When the sample-forming material is too large, adequate infiltration of the organic compound (X) becomes difficult to be achieved, and the rate of successful determination of the molecular structure tends to decrease.

The used amount of the sample-forming material is, for example, from 0.1 to 50 mg, preferably from 0.5 to 15 mg, and particularly preferably from 1 to 3 mg.

The metal complex crystal included in the sample-forming material has excellent toughness. Thus, the cavities of the metal complex crystal may be filled with a solvent or may be not filled with a solvent.

The method of allowing the organic compound (X) to infiltrate into the sample-forming material and fixing the organic compound (X) in cavities of the sample-forming material can be properly selected depending on the state of the organic compound (X) (liquid, gas, or solid).

When the organic compound (X) is a liquid, examples of the fixing method include a method including placing the sample-forming material in a container, such as a test tube, and then adding the organic compound (X) to the container and allowing the container to stand still at -20 to 100°C for 0.25 to 24 hours.

When the organic compound (X) is a gas, examples of the fixing method include a method including filling an airtight container, such as a test tube with a cap, with an inert gas (e.g., helium gas or nitrogen gas) and then enclosing the sample-forming material and the organic compound (X) in the airtight container and allowing the airtight container to stand still at -20 to 100°C for 0.25 to 24 hours. Furthermore, before the enclosing in the airtight container, a sample gas containing the gaseous organic compound (X) may be subjected to a treatment for increasing the organic compound (X) concentration, for example, a separation treatment by gas chromatography.

When the organic compound (X) is a solid, examples of the fixing method include a method including placing the sample-forming material in a container, such as a test tube, and then adding a solution having the organic compound (X) dissolved in a solvent inactive to the organic compound (X) (hereinafter, may be referred to as "inert solvent") to the container, and allowing the container to stand still at -20 to 100°C for 0.25 to 24 hours. Furthermore, after the standing, a concentration treatment of the infiltrated solution may be performed. By performing the concentration treatment, the rate of fixing the organic compound (X) in the cavities is increased, and the rate of successful determination of a molecular structure is improved.

The sample-forming material has excellent solvent resistance, and the crystal structure can be maintained in various solvents. Thus, among inert solvents, a solvent that improves the rate of successful determination of a molecular structure can be selected and used. Examples of the inert solvent include alcohols such as methanol, ethanol, and isopropyl alcohol; and acyclic or cyclic ethers such as tetrahydrofuran (THF), dimethoxyethane, and dioxane. One type of these may be used alone, or two or more types thereof may be used in combination.

The organic compound (X) concentration in the solution obtained by dissolving the organic compound (X) in the inert solvent is, for example, from 0.001 to 50 µg/µL, preferably from 0.01 to 5 µg/µL, and more preferably from 0.1 to 1 µg/µL.

The sample-forming material includes an amide group, and thus, when the organic compound (X) is allowed to infiltrate into the sample-forming material, the organic compound (X) incorporated into the cavities of the sample-forming material can be firmly fixed by a hydrogen bond.

Through this step, an X-ray crystallographic sample obtained by fixing the organic compound (X) in the cavities of the sample-forming material is obtained.

### (Step 2)

The step 2 is a step of irradiating the X-ray crystallographic sample obtained in the step described above (i.e., material obtained by fixing the organic compound (X) in the cavities of the sample-forming material) with X-rays, analyzing obtained diffraction data, and determining a molecular structure of the organic compound (X).

In the method for determining the molecular structure of the organic compound (X), the organic compound (X) is not required to be crystallized, and thus, even when the amount of the organic compound (X) is too small to yield a single crystal or even when the organic compound (X) is a compound that cannot form a single crystal, the molecular structure can be determined by X-ray crystallography.

In addition, according to the method for determining the molecular structure of the organic compound (X), even when the organic compound (X) is a gas, the molecular structure can be determined by X-ray crystallography.

Thus, the method for determining the molecular structure of the organic compound (X) can easily determine a structure of a trace amount of an impurity in pharmaceuticals, a substance that causes an odor (including smell) (e.g., fragrance), a food additive, and a trace amount of a component in animals and plants, for example.

### Method for Determining Molecular Structure of Gaseous Odor Component (Y)

The method for determining the molecular structure of the gaseous odor component (Y) includes the following step 1, step 2, and step 3.
step 1: feeding a sample gas containing the gaseous odor component (Y) to a column for separating gas components and fractionating the gaseous odor component (Y);
Step 2: allowing the fractionated gaseous odor component (Y) to infiltrate into the sample-forming material (or the metal complex crystal), and fixing the gaseous odor component (Y) in cavities of the sample-forming material (or the metal complex crystal).
Step 3: irradiating the sample-forming material (or the metal complex crystal)containing the fixed gaseous odor component (Y) with X-rays, analyzing obtained diffraction data, and determining a molecular structure of the gaseous odor component (Y).

### (Step 1)

The step 1 is a step of feeding a sample gas containing the gaseous odor component (Y) to a column for separating gas components and fractionating the gaseous odor component (Y). The step 1 is a step of feeding a sample gas containing the gaseous odor component (Y) together with a carrier gas to a column for separating gas components and fractionating the gaseous odor component (Y) together with the carrier gas.

The gaseous odor component (Y) is a gaseous organic compound emitting an odor and is preferably a compound having a functional group and molecular weight similar to those of the organic compound (X) described above.

The sample gas may contain two or more types of odor components (Y1, Y2, ..., Yn; n represents an integer of 2 or greater) and also may contain a gas component other than the odor component(s).

The column for separating gas components includes a stationary phase inside the column, and has a function of separating the respective components by utilizing the fact that, when a sample gas containing a plurality of components is supplied in the column together with a carrier gas, the respective components in the sample gas have interactions with the stationary phase, the interactions being different from each other, and thus, the retention time is varied for the respective components. The carrier gas is preferably an inert gas such as helium gas or nitrogen gas.

The column may be connected with a device for fractionating the sample gas separated in the column.

When the sample gas contains two or more types of gaseous odor components (Y1, Y2, ..., Yn; n represents an integer of 2 or greater), it is preferable to separately fractionate the gaseous odor component (Y1), the gaseous odor component (Y2), ..., and the gaseous odor component (Yn). Furthermore, the gaseous odor component may be fractionated together with the carrier gas.

### (Step 2)

The step 2 is a step of allowing the gaseous odor component (Y) fractionated in the step 1 to infiltrate into the sample-forming material, and fixing the gaseous odor component (Y) in cavities of the sample-forming material.

This step can be performed by the same method as the step 1 of the method for determining the molecular structure of the organic compound (X). Through this step, an X-ray crystallographic sample obtained by fixing the gaseous odor component (Y) in the cavities of the metal complex crystal, is obtained.

### Step 3

The step 3 is a step of irradiating the X-ray crystallographic sample obtained in the step described above (i.e., material obtained by fixing the gaseous odor component (Y) in the cavities of the sample-forming material) with X-rays, analyzing obtained diffraction data, and determining a molecular structure of the gaseous odor component (Y).

This step can be performed by the same method as the step 2 of the method for determining the molecular structure of the organic compound (X).

The method for determining the molecular structure of the gaseous odor component (Y) can form an X-ray crystallographic sample by fixing the gaseous odor component (Y) to the sample-forming material while the gaseous odor component (Y) is maintained in a gaseous state, and, the molecular structure can be easily determined by subjecting the formed X-ray crystallographic sample to X-ray crystallography. In addition, for the substance that causes an odor (including smell), in the related art, the odor component has been identified depending on human sensibilities by the GC-MS-olfactometry method; and however, according to the method for determining the molecular structure of the gaseous odor component (Y), the molecular structure of the gaseous odor component (Y) can be identified accurately without depending on human sensibilities.

The configurations and their combinations of the present disclosure described above are merely examples, and addition, omission, substitution, and modification of the configurations may be appropriately made without departing from the gist of the present disclosure. Each embodiment disclosed in the present specification can be combined with any other feature disclosed herein. Furthermore, the present disclosure is not limited to the embodiments. Examples

The present disclosure will next be described in more detail by way of Examples, but the present disclosure is not limited to Examples.

The single crystal X-ray crystallography was performed by using the following automated single crystal X-ray diffraction system.
Automated single crystal X-ray diffraction system: "XtaLAB SynergyCustom", available from Rigaku Holdings Corporation
Ray source: Cu-Kα line (wavelength: 0.79 Å)
Output: 50 mA, 24 kV

### Example 1 (Production of X-ray Crystallographic Sample-Forming Material Containing DMF and Water in Cavities)

In a 10 mL screw-cap test tube, a carboxylic acid-based ligand represented by Formula (c2-1-1) below (24.2 mg, 0.1 mol), a pyridine-based ligand represented by Formula (p2-1) below (31.8 mg, 0.1 mol), Zn(NO₃)₂6H₂O (29.7 mg, 0.1 mol), and a DMF/H₂O (4 mL/1 mL) mixed solvent were charged and dissolved by ultrasonic treatment.

Subsequently, the test tube was capped tightly and allowed to stand still at 100°C for 6 hours.

A procedure, in which the solvent in the test tube was removed by using a Pasteur pipette and replaced with a new DMF/H₂O (4 mL/1 mL) mixed solvent, was repeated for 3 times.

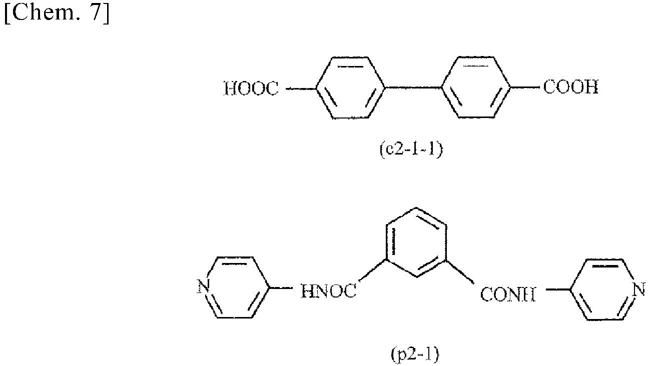

Thus, needle-like or block-like single crystal particles having transparency and having lengths of 10 to 200 µm were obtained. These particles were designated as a sample-forming material 1. The sample-forming material 1 contains DMF and water in the cavities.

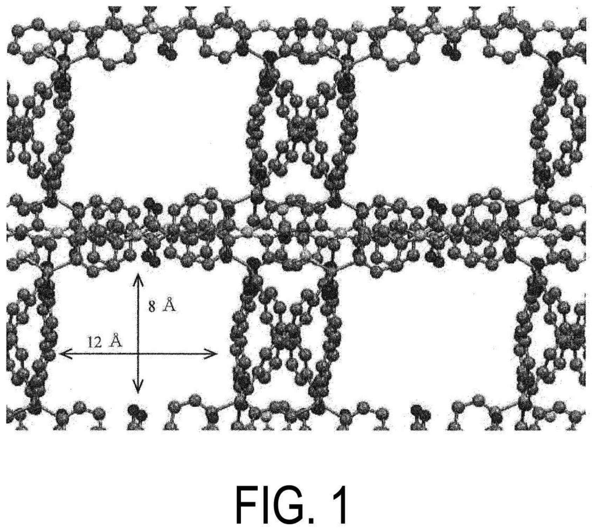

The crystal structure of the obtained sample-forming material 1 was confirmed by subjecting the obtained sample-forming material 1 to single crystal X-ray crystallography. The schematic view of the sample-forming material 1 is illustrated in FIG. 1.

The single crystal X-ray crystallography confirmed that the sample-forming material 1 had a three-dimensional network structure.

Furthermore, the inscribed ellipse of each of the cavities included in the sample-forming material 1 had a major axis of 12 Å and a minor axis of 8 Å.

### Example 2 (Production of X-ray Crystallographic Sample-Forming Material 2 Containing Ethyl Acetate in Cavities)

From a screw-cap test tube containing DMF/H₂O (4 mL/1 mL) and the sample-forming material 1 containing DMF in the cavities obtained by the method same as in Example 1, the solvent was removed and replaced with an ethyl acetate/DMF (1 mL/4 mL) mixed solvent, and the mixture was allowed to stand still for 1 day.

Subsequently, the solvent in the test tube was removed and replaced with an ethyl acetate/DMF (1 mL/2 mL) mixed solvent, and the mixture was allowed to stand still for 1 day.

Next, the solvent in the test tube was removed and replaced with an ethyl acetate/DMF (1 mL/1 mL) mixed solvent, and the mixture was allowed to stand still for 1 day.

Furthermore, the solvent in the test tube was removed and replaced with an ethyl acetate/DMF (2 mL/1 mL) mixed solvent, and the mixture was allowed to stand still for 1 day.

Subsequently, a procedure, in which the solvent in the test tube was removed and replaced with ethyl acetate, was repeated for 3 times.

Thus, needle-like or block-like single crystal particles having ethyl acetate in the cavities were obtained. These particles were designated as a sample-forming material 2. The sample-forming material 2 contains ethyl acetate in the cavities.

When the single crystal X-ray crystallography was performed for the obtained sample-forming material 2, a three-dimensional network structure similar to that of the sample-forming material 1 could be confirmed.

### Example 3 (Production of X-ray Crystallographic Sample-Forming Material 3 Containing THF in Cavities)

From a screw-cap test tube containing ethyl acetate and the sample-forming material 2 containing ethyl acetate in the cavities obtained by the method same as in Example 2, the solvent was removed and replaced with a THF/ethyl acetate (1 mL/4 mL) mixed solvent, and the mixture was allowed to stand still for 1 day.

Subsequently, the solvent in the test tube was removed and replaced with a THF/ethyl acetate (1 mL/2 mL) mixed solvent, and the mixture was allowed to stand still for 1 day.

Next, the solvent in the test tube was removed and replaced with a THF/ethyl acetate (1 mL/1 mL) mixed solvent, and the mixture was allowed to stand still for 1 day.

Furthermore, the solvent in the test tube was removed and replaced with a THF/ethyl acetate (2 mL/1 mL) mixed solvent, and the mixture was allowed to stand still for 1 day.

Subsequently, a procedure, in which the solvent in the test tube was removed and replaced with THF, was repeated for 3 times.

Thus, needle-like or block-like single crystal particles having THF in the cavities were obtained. These particles were designated as a sample-forming material 3. The sample-forming material 3 contains THF in the cavities.

When the single crystal X-ray crystallography was performed for the obtained sample-forming material 3, a three-dimensional network structure similar to that of the sample-forming material 1 was confirmed.

### Comparative Example 1 (Production of X-ray Crystallographic Sample-Forming Material 4)

First, in a 50 mL vial, 2,4,6-tris(4-pyridyl)-1,3,5-triazine (TPT) (31.3 mg, 0.1 mmol), 21 mL of CHCl₃, and 1.7 mL of methanol were added and completely dissolved by ultrasonic treatment. In a screw-cap test tube, 4.5 mL of the solution thus obtained was charged.

Then, in a 10 mL vial, zinc chloride (20.5 mg, 0.15 mmol) and 5 mL of methanol were added and dissolved by ultrasonic treatment. In the screw-cap test tube, 1 mL of the solution thus obtained was added.

Then, the screw-cap test tube was capped and allowed to stand still at room temperature for 3 days. Thus, transparent needle-like or block-like single crystal particles having lengths of 10 to 200 µm were obtained. This was designated as a sample-forming material 4.

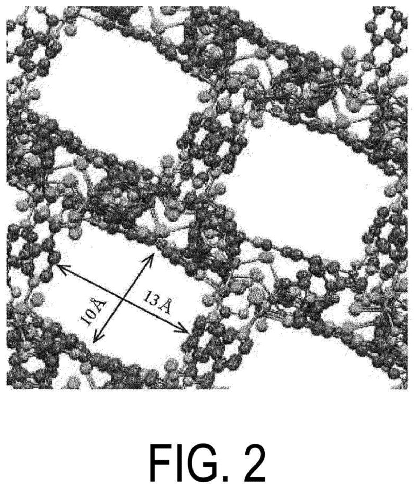

The single crystal X-ray crystallography of the sample-forming material 4 was performed. The schematic view of the sample-forming material 4 is illustrated in FIG. 2.

By the single crystal X-ray crystallography, it was found that the inscribed ellipse of each of the cavities included in the sample-forming material 4 had a major axis of 13 Å and a minor axis of 10 Å.

### Solvent Resistance Evaluation

Solvent resistance was evaluated for the sample-forming material 1 obtained in Example 1 and the sample-forming material 4 obtained in Comparative Example 1 by the following method.

In a microvial, a single particle of single crystal particle of each of the sample-forming materials was charged, 1 µL of the following solvent was added to the microvial and the mixture was allowed to stand still at 50°C or room temperature (25°C) for 17 hours, and whether the crystal structure was maintained or not after the standing was visually observed. Then, the solvent resistance was evaluated based on the following criteria.

### Solvent Resistance Evaluation Criteria

Excellent (○): The crystal structure could be maintained, and the framework of the metal complex could be observed by the single crystal X-ray measurement.

Good (Δ): The crystal structure could not be not maintained in the case of the standing at 50°C for 17 hours; and however, the crystal structure could be maintained in the case of the standing at room temperature for 17 hours.

Poor (×): The crystal structure could not be maintained.

### [Table 1]

**Table 1**

| Solvent | Sample forming material 1 | Sample forming material 4 |
|---|---|---|
| Water | △ | × |
| DMSO | ○ | × |
| METHANOL | ○ | × |
| Acetonitrile | ○ | × |
| Acetone | ○ | ○ |
| Ethyl acetate | ○ | ○ |
| Chloroform | ○ | ○ |

### Example 11 (Determination of Structure of Organic Compound Having Ketone Group)

In a microvial with a septum cap, 45 µL of THF was charged, and then a single particle of the single crystal particle of the sample-forming material 1 containing DMF obtained in Example 1 was immersed in the THF. Subsequently, the THF was removed as much as possible using a pipette.

Next, 1 µg of 4-methylacetophenone was added in the microvial. Thereafter, the microvial was capped and allowed to stand still in a thermostatic chamber at 50°C for 17 hours.

Subsequently, the sample-forming material 1 was removed from the microvial. The sample-forming material 1 removed from the microvial was used as an X-ray crystallographic sample.

The obtained X-ray crystallographic sample was mounted on an X-ray diffractometer and analyzed for the crystal structure, and the ability to be analyzed for the crystal structure was evaluated based on the following evaluation criteria. The result is presented in the following table.

### Evaluation Criteria for Ability to be analyzed for Crystal Structure

⊚ (Good): The molecular structure could be determined very clearly.
∘ (Fair): The molecular structure could be determined with a certain degree of accuracy.
× (Failed): The molecular structure could not be determined.

### Examples 12 to 14 (Determination of Structure of Organic Compound Having Ketone Group)

The same procedure as in Example 11 was performed except for using each of the organic compounds (X) listed in Table 2 below in place of 4-methylacetophenone. The result is presented in the following table.

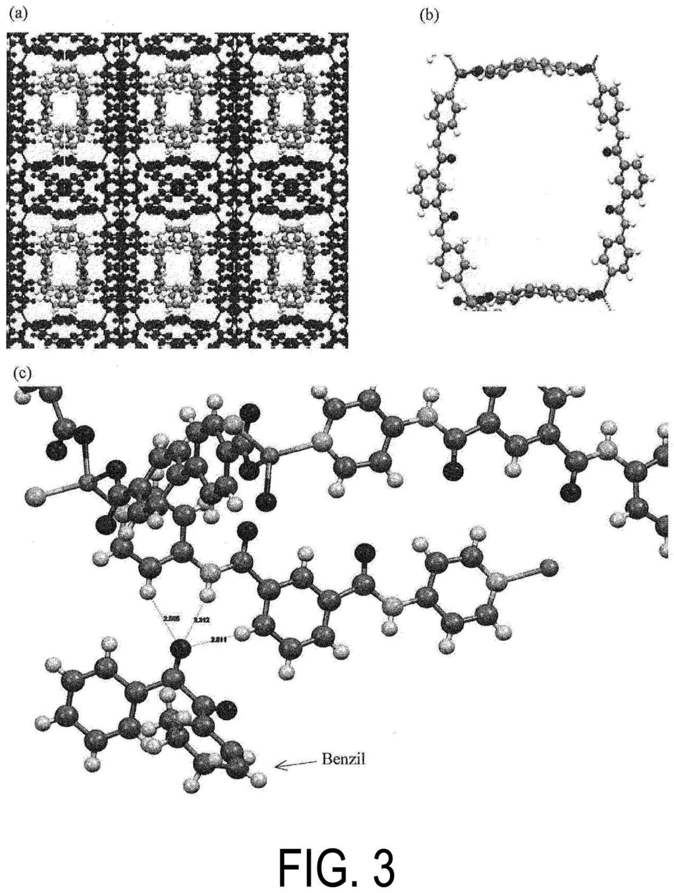

Furthermore, FIG. 3(a) illustrates a schematic view of a crystal structure of an X-ray crystallographic sample (metal complex crystal in which benzil was fixed in cavities) obtained in Example 14. FIG. 3(b) illustrates a schematic view of a shape of a cavity of the X-ray crystallographic sample. FIG. 3(c) illustrates an interaction between N-H comprised in an amide group of the sample-forming material 1 and an oxygen atom of an oxo group contained in the benzil.

### Comparative Examples 11 to 14 (Determination of Structure of Organic Compound Having Ketone Group)

The same procedure as in Examples 11 to 14 was performed except for using the sample-forming material 4 in place of the sample-forming material 1. The result is presented in the following table.

**[Table 2]**

| Table 2 | | | | |
|---|---|---|---|---|
| No. | Organic compound (X) | log P value | Sample forming material 1 | Sample forming material 4 |
| 11 | 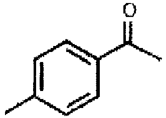 | 2.10 | ⊚ | ○ |
| 12 | 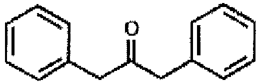 | 3.18 | ⊚ | × |
| 13 | 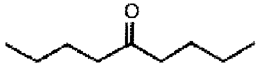 | 2.88 | ⊚ | × |
| 14 | 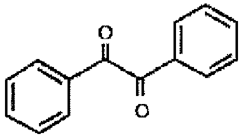 | 3.38 | ⊚ | Not performed |

### Examples 15 to 27 and Comparative Examples 15 to 27

The same procedure as Example 11 was performed except for using each of the organic compounds (X) listed in Tables 3 to 5 below in place of 4-methylacetophenone, and thus each X-ray crystallographic sample was obtained and subjected to crystallography. The result is presented in the following table.

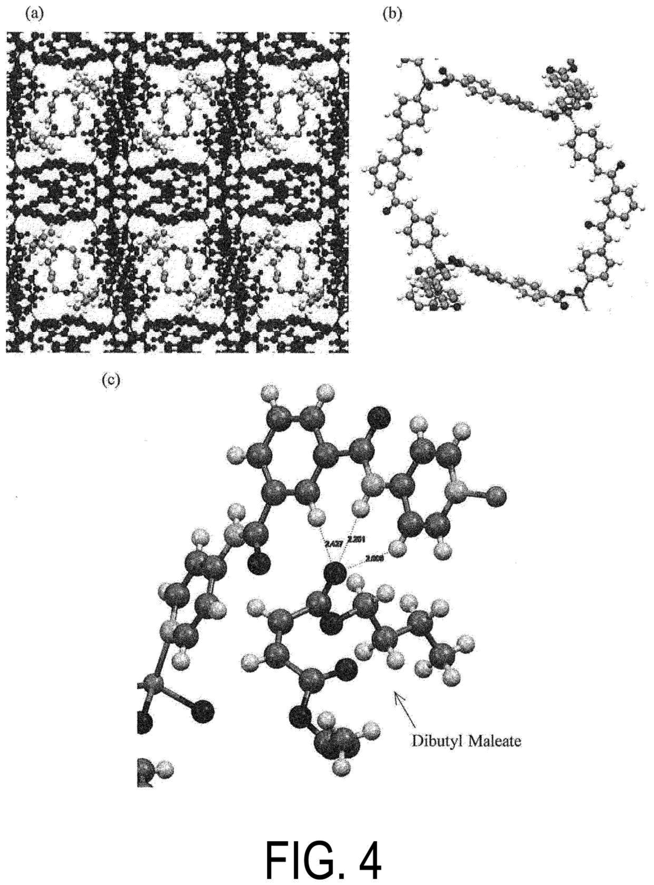

Furthermore, FIG. 4(a) illustrates a schematic view of a crystal structure of an X-ray crystallographic sample (metal complex crystal in which dibutyl fumarate was fixed in cavities) obtained in Example 22. FIG. 4(b) illustrates a schematic view of a shape of a cavity of the X-ray crystallographic sample. FIG. 4(c) illustrates an interaction between N-H comprised in an amide group of the sample-forming material 1 and an oxygen atom of an oxo group contained in the dibutyl fumarate.

**[Table 3]**

| Table 3 | | | | |
|---|---|---|---|---|
| No. | Organic compound (X) | log P value | Sample forming material 1 | Sample forming material 4 |
| 15 | 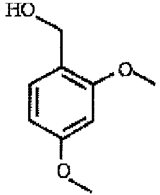 | 1.24 | ⊚ | ○ |
| 16 | 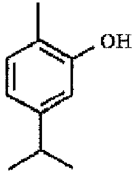 | 3.49 | ⊚ | ⊚ |
| 17 | 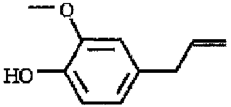 | 2.27 | ⊚ | ⊚ |
| 18 | 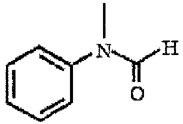 | 1.09 | ⊚ | × |

**[Table 4]**

| Table 4 | | | | |
|---|---|---|---|---|
| No. | Organic compound (X) | log P value | Sample forming material 1 | Sample forming material 4 |
| 19 | 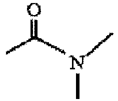 | -0.77 | ⊚ | × (Collapse of Crystal structure) |
| 20 | 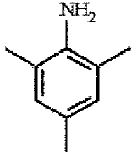 | 2.72 | ⊚ | × (Collapse of Crystal structure) |
| 21 | 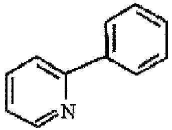 | 2.63 | ○ | × (Collapse of Crystal structure) |
| 22 | 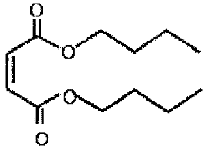 | 4.16 | ⊚ | ○ |
| 23 | 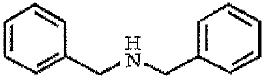 | 2.67 | ○ | × (Collapse of Crystal structure) |

**[Table 5]**

| Table 5 | | | | |
|---|---|---|---|---|
| No. | Organic compound (X) | log P value | Sample forming material 1 | Sample forming material 4 |
| 24 | 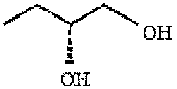 | -0.29 | ⊚ | × (Collapse of Crystal structure) |
| 25 | 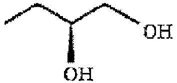 | -0.29 | ⊚ | × (Collapse of Crystal structure) |
| 26 | 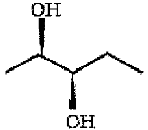 | -0.92 | ⊚ | × (Collapse of Crystal structure) |
| 27 | 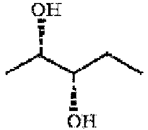 | -0.92 | ⊚ | × (Collapse of Crystal structure) |

### Example 28 (Determination of Structure of Odor Component)

In a vial containing nitrobenzene, 5 mL of a gas in the gas phase of the vial was fractionated by suction using a syringe, and this fractionated gas was used as a gaseous odor component (Y).

To a vial with a cap, the vial being charged with a single particle of the single crystal particle of the sample-forming material 1, the fractionated gaseous odor component (Y) was added and the vial was allowed to stand still at 50°C for 2 hours.

Subsequently, the sample-forming material 1 was removed from the vial and used as an X-ray crystallographic sample.

When the obtained X-ray crystallographic sample was mounted on an X-ray diffractometer and analyzed for the crystal structure, the entire structure of the molecule could be determined by an X-ray diffraction photograph.

### Example 29 (Determination of Structure of Odor Component)

In 1 mL of acetone, 5 mg of nitrobenzene as an odor component was dissolved, and thus a sample solution was obtained.

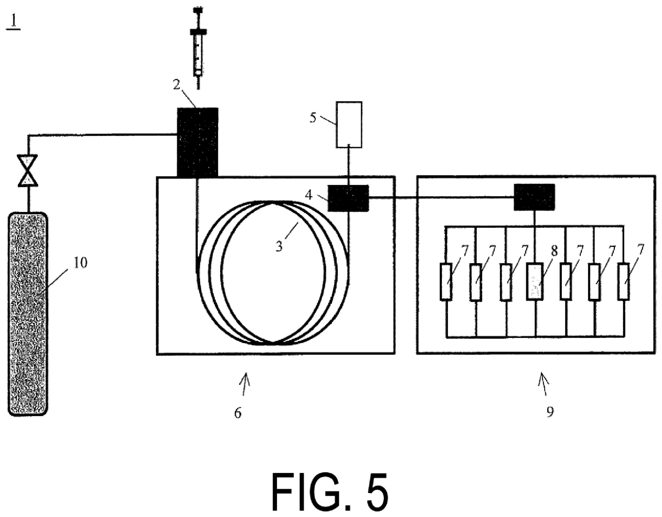

From an inlet 2 of a gas chromatograph-fractionation system having the following specification, 5 µL of the obtained sample solution was injected, the component passed through a column 3 was introduced from a branching part into a fractionating equipment 6. The component was fractioned into 6 tubes of fractionation tubes 7 (fractionation tubes cooled to -70°C), each tube containing a single particle of the single crystal particle of the sample-forming material 1, and the fractionation tubes 7 were allowed to stand still at 50°C for 17 hours (see FIG. 5).

Thereafter, the sample-forming material 1 charged in each of the fractionation tubes 7 was removed and used as an X-ray crystallographic sample.

When the obtained X-ray crystallographic sample was mounted on an X-ray diffractometer and analyzed for the crystal structure, the entire structure of the molecule could be determined by an X-ray diffraction photograph.

### Specification of Gas Chromatograph-Fractionation System

Gas chromatograph-fractionation system: Product name "VPS-2800", available from GL Sciences Inc.
Column: DB-5MS 30 m-0.32 mm-0.5 µm, available from GL Sciences Inc.
Column heating temperature: 80°C (0 min) → 2°C/min → 140°C (0 min)
Carrier Gas: helium
Column flow rate: 6.58 mL/min

### Example 30 (Determination of Structure of Odor Component)

The same procedure as in Example 29 was performed except for using α-terpineol in place of nitrobenzene. As a result, the molecular structure of the α-terpineol was determined by an X-ray diffraction photograph.

### Example 31 (Determination of Structure of Odor Component)

The same procedure as in Example 29 was performed except for using eugenol in place of nitrobenzene. As a result, the molecular structure of the eugenol was determined by an X-ray diffraction photograph.

To summarize the above, configurations and variations of the present disclosure are additionally described below.
[1] An X-ray crystallographic sample-forming material,
   the material being a material for forming an X-ray crystallographic sample containing an organic compound in a regularly arranged state, and
   the material containing a metal complex crystal described below:
      Metal complex crystal: The metal complex crystal being a metal complex crystal containing a metal ion and a ligand coordinated to the metal ion, the metal complex crystal having a three-dimensional network structure having regularly arranged cavities, the ligand containing a carboxylic acid-based ligand (c) and a pyridine-based ligand (p), and the carboxylic acid-based ligand (c) and the pyridine-based ligand (p) being in a combination of the following [I] or [II]:
      [I] A compound represented by Formula (c1) and a compound represented by Formula (p1); or
      [II] A compound represented by Formula (c2) and a compound represented by Formula (p2)
[2] The X-ray crystallographic sample-forming material according to [1], wherein the metal ion is a metal ion selected from a zinc ion, an iron ion, a cobalt ion, a nickel ion, a copper ion, or a silver ion.
[3] The X-ray crystallographic sample-forming material according to [1] or [2], wherein the organic compound is an organic compound including a functional group capable of interacting with at least one group selected from a CONH group, a CO group, and an NH group.
[4] The X-ray crystallographic sample-forming material according to [1] or [2], wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, and a sulfonamide group.
[5] The X-ray crystallographic sample-forming material according to any one of [1] to [4], wherein the organic compound is an organic compound having a log P value of -5 or greater and 7 or less.
[6] The X-ray crystallographic sample-forming material according to any one of [1] to [5], wherein the compound represented by Formula (c1) in [I] is at least one compound selected from a compound represented by Formula (c1-1), a compound represented by Formula (c1-2), a compound represented by Formula (c1-3), and a compound represented by Formula (c1-4).
[7] The X-ray crystallographic sample-forming material according to any one of [1] to [5], wherein the compound represented by Formula (c1) in [I] is the compound represented by Formula (c1-1).
[8] The X-ray crystallographic sample-forming material according to any one of [1] to [7], wherein the compound represented by Formula (p1) in [I] is at least one compound selected from a compound represented by Formula (p1-1), a compound represented by Formula (p1-2), a compound represented by Formula (p1-3), a compound represented by Formula (p1-4), a compound represented by Formula (p1-5), a compound represented by Formula (p1-6), and a compound represented by Formula (p1-7).
[9] The X-ray crystallographic sample-forming material according to any one of [1] to [7], wherein the compound represented by Formula (p1) in [I] is at least one compound selected from compounds represented by Formula (p1-1), Formula (p1-2), Formula (p1-5), and Formula (p1-6).
[10] The X-ray crystallographic sample-forming material according to any one of [1] to [7], wherein the compound represented by Formula (p1) in [I] is the compound represented by Formula (p1-1) and/or the compound represented by Formula (p1-2).
[11] The X-ray crystallographic sample-forming material according to any one of [1] to [10], wherein the compound represented by Formula (c2) in [II] is at least one compound selected from a compound represented by Formula (c2-1), a compound represented by Formula (c2-2), a compound represented by Formula (c2-3), a compound represented by Formula (c2-4), and a compound represented by Formula (c2-5).
[12] The X-ray crystallographic sample-forming material according to any one of [1] to [10], wherein the compound represented by Formula (c2) in [II] is the compound represented by Formula (c2-1) and/or the compound represented by Formula (c2-2).
[13] The X-ray crystallographic sample-forming material according to any one of [1] to [10], wherein the compound represented by Formula (c2) in [II] is the compound represented by Formula (c2-1).
[14] The X-ray crystallographic sample-forming material according to any one of [1] to [13], wherein the compound represented by Formula (p2) in [II] is at least one compound selected from a compound represented by Formula (p2-1), a compound represented by Formula (p2-2), a compound represented by Formula (p2-3), and a compound represented by Formula (p2-4).
[15] The X-ray crystallographic sample-forming material according to any one of [1] to [13], wherein the compound represented by Formula (p2) in [II] is the compound represented by Formula (p2-1).
[16] The X-ray crystallographic sample-forming material according to any one of [1] to [15], wherein a content ratio of the carboxylic acid-based ligand (c) to the pyridine-based ligand (p) [carboxylic acid-based ligand (c)/pyridine-based ligand (p); molar ratio] is from 30/70 to 70/30.
[17] The X-ray crystallographic sample-forming material according to any one of [1] to [16], wherein a content ratio of the metal ion to the carboxylic acid-based ligand (c) [metal ion/carboxylic acid-based ligand (c); molar ratio] is from 30/70 to 70/30.
[18] The X-ray crystallographic sample-forming material according to any one of [1] to [17], wherein a content ratio of the metal ion to the pyridine-based ligand (p) [metal ion/pyridine-based ligand (p); molar ratio] is from 30/70 to 70/30.
[19] The X-ray crystallographic sample-forming material according to any one of [1] to [18], wherein a proportion of a total content of the carboxylic acid-based ligand (c) and the pyridine-based ligand (p) with respect to a total amount of the ligands comprised in the metal complex crystal is 50 wt.% or greater.
[20] A method for determining a molecular structure of an organic compound (X), the method including the following steps 1 and 2:
   step 1: allowing the organic compound (X) to infiltrate into the X-ray crystallographic sample-forming material described in any one of [1] to [19], and fixing the organic compound (X) in cavities of the X-ray crystallographic sample-forming material; and
   step 2: irradiating the X-ray crystallographic sample-forming material containing the fixed organic compound (X) with X-rays, analyzing obtained diffraction data, and determining a molecular structure of the organic compound (X).
[21] A method for determining a molecular structure of a gaseous odor component (Y), the method including the following steps 1, 2, and 3:
   step 1: feeding a sample gas containing the gaseous odor component (Y) to a column for separating gas components and fractionating the gaseous odor component (Y);
   step 2: allowing the fractionated gaseous odor component (Y) to infiltrate into the X-ray crystallographic sample-forming material described in any one of [1] to [19], and fixing the gaseous odor component (Y) in cavities of the X-ray crystallographic sample-forming material; and
   step 3: irradiating the X-ray crystallographic sample-forming material containing the fixed gaseous odor component (Y) with X-rays, analyzing obtained diffraction data, and determining a molecular structure of the gaseous odor component (Y).
[22] Use of the following metal complex crystal as an X-ray crystallographic sample-forming material for an organic compound:
   Metal complex crystal: The metal complex crystal being a metal complex crystal containing a metal ion and a ligand coordinated to the metal ion, the metal complex crystal having a three-dimensional network structure having regularly arranged cavities, the ligand containing a carboxylic acid-based ligand (c) and a pyridine-based ligand (p), and the carboxylic acid-based ligand (c) and the pyridine-based ligand (p) being in a combination of the following [I] or [II]:
   [I] A compound represented by Formula (c1) and a compound represented by Formula (p1)
   [II] A compound represented by Formula (c2) and a compound represented by Formula (p2)
[23] The use as an X-ray crystallographic sample-forming material for an organic compound according to [22], wherein the metal ion is a metal ion selected from a zinc ion, an iron ion, a cobalt ion, a nickel ion, a copper ion, or a silver ion.
[24] The use as an X-ray crystallographic sample-forming material for an organic compound according to [22] or [23], wherein the organic compound is an organic compound including a functional group capable of interacting with at least one group selected from a CONH group, a CO group, and an NH group.
[25] The use as an X-ray crystallographic sample-forming material for an organic compound according to [22] or [23], wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.
[26] The use as an X-ray crystallographic sample-forming material for an organic compound according to any one of [22] to [25], wherein the organic compound is an organic compound having a log P value of -5 or greater and 7 or less.
[27] The use as an X-ray crystallographic sample-forming material for an organic compound according to any one of [22] to [26], wherein the compound represented by Formula (c1) in [I] is at least one compound selected from a compound represented by Formula (c1-1), a compound represented by Formula (c1-2), a compound represented by Formula (c1-3), and a compound represented by Formula (c1-4).
[28] The use as an X-ray crystallographic sample-forming material for an organic compound according to any one of [22] to [26], wherein the compound represented by Formula (c1) in [I] is the compound represented by Formula (c1-1).
[29] The use as an X-ray crystallographic sample-forming material for an organic compound according to any one of [22] to [28], wherein the compound represented by Formula (p1) in [I] is at least one compound selected from a compound represented by Formula (p1-1), a compound represented by Formula (p1-2), a compound represented by Formula (p1-3), a compound represented by Formula (p1-4), a compound represented by Formula (p1-5), a compound represented by Formula (p1-6), and a compound represented by Formula (p1-7).
[30] The use as an X-ray crystallographic sample-forming material for an organic compound according to any one of [22] to [28], wherein the compound represented by Formula (p1) in [I] is at least one compound selected from the compounds represented by Formula (p1-1), Formula (p1-2), Formula (p1-5), and Formula (p1-6).
[31] The use as an X-ray crystallographic sample-forming material for an organic compound according to any one of [22] to [28], wherein the compound represented by Formula (p1) in [I] is the compound represented by Formula (p1-1) and/or the compound represented by Formula (p1-2).
[32] The use as an X-ray crystallographic sample-forming material for an organic compound according to any one of [22] to [31], wherein the compound represented by Formula (c2) in [II] is at least one compound selected from a compound represented by Formula (c2-1), a compound represented by Formula (c2-2), a compound represented by Formula (c2-3), a compound represented by Formula (c2-4), and a compound represented by Formula (c2-5).
[33] The use as an X-ray crystallographic sample-forming material for an organic compound according to any one of [22] to [31], wherein the compound represented by Formula (c2) in [II] is the compound represented by Formula (c2-1) and/or the compound represented by Formula (c2-2).
[34] The use as an X-ray crystallographic sample-forming material for an organic compound according to any one of [22] to [31], wherein the compound represented by Formula (c2) in [II] is the compound represented by Formula (c2-1).
[35] The use as an X-ray crystallographic sample-forming material for an organic compound according to any one of [22] to [34], wherein the compound represented by Formula (p2) in [II] is at least one compound selected from a compound represented by Formula (p2-1), a compound represented by Formula (p2-2), a compound represented by Formula (p2-3), and a compound represented by Formula (p2-4).
[36] The use as an X-ray crystallographic sample-forming material for an organic compound according to any one of [22] to [34], wherein the compound represented by Formula (p2) in [II] is the compound represented by Formula (p2-1).
[37] The use as an X-ray crystallographic sample-forming material for an organic compound according to any one of [22] to [36], wherein a content ratio of the carboxylic acid-based ligand (c) to the pyridine-based ligand (p) [carboxylic acid-based ligand (c)/pyridine-based ligand (p); molar ratio] is from 30/70 to 70/30.
[38] The use as an X-ray crystallographic sample-forming material for an organic compound according to any one of [22] to [37], wherein a content ratio of the metal ion to the carboxylic acid-based ligand (c) [metal ion/carboxylic acid-based ligand (c); molar ratio] is from 30/70 to 70/30.
[39] The use as an X-ray crystallographic sample-forming material for an organic compound according to any one of [22] to [38], wherein a content ratio of the metal ion to the pyridine-based ligand (p) [metal ion/pyridine-based ligand (p); molar ratio] is from 30/70 to 70/30.
[40] The use as an X-ray crystallographic sample-forming material for an organic compound according to any one of [22] to [39], wherein a proportion of a total content of the carboxylic acid-based ligand (c) and the pyridine-based ligand (p) with respect to a total amount of the ligands comprised in the metal complex crystal is 50 wt.% or greater.

### Industrial Applicability

When the X-ray crystallographic sample-forming material of the present disclosure is used, even in the case where the amount of the organic compound (X) is too small to form a single crystal or even in the case of a compound that cannot form a single crystal, and even when the organic compound (X) is any of solid, liquid, or gas, the molecular structure can be determined by X-ray crystallography.

### Reference Signs List

1 Gas chromatograph-fractionation system
2 Inlet
3 Column
4 Branching part
5 Detector
6 Column heating tank
7 Fractionation tube
8 Discharge line
9 Fractionation tube cooling tank
10 Carrier gas

## Claims

1. An X-ray crystallographic sample-forming material,
the material being a material for forming an X-ray crystallographic sample containing an organic compound in a regularly arranged state, and
the material comprising a metal complex crystal:
Metal complex crystal: The metal complex crystal being a metal complex crystal containing a metal ion and a ligand coordinated to the metal ion, the metal complex crystal having a three-dimensional network structure having regularly arranged cavities, the ligand containing a carboxylic acid-based ligand (c) and a pyridine-based ligand (p), and the carboxylic acid-based ligand (c) and the pyridine-based ligand (p) being in a combination of [I] or [II]:
[I] a compound represented by Formula (c1) and a compound represented by Formula (p1); or
[II] a compound represented by Formula (c2) and a compound represented by Formula (p2) 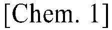 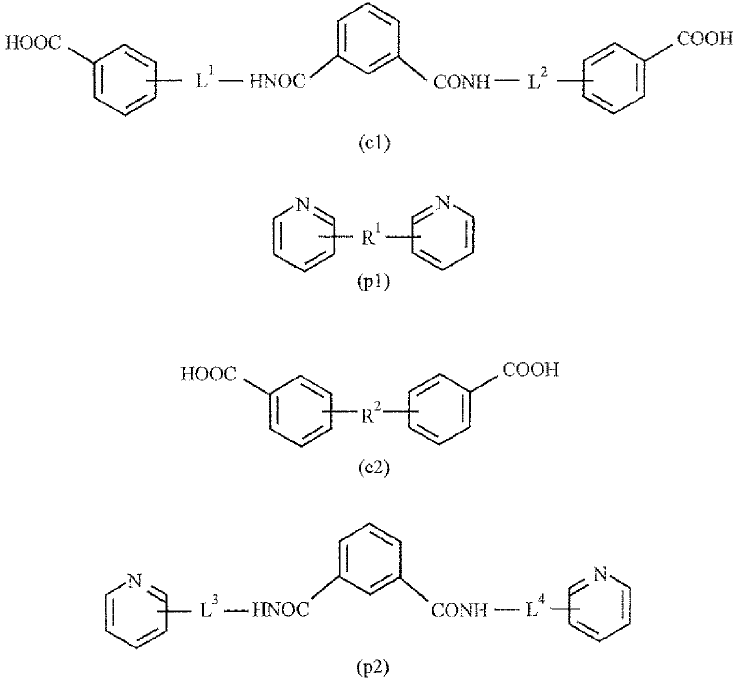
where R¹ and R² are the same or different and each represent a group selected from a single bond, a divalent hydrocarbon group, a divalent heterocyclic group, or a divalent group containing two or more of the groups, the two or more of the groups being linked, L¹ to L⁴ are the same or different and each represent a single bond or a linking group.

2. The X-ray crystallographic sample-forming material according to claim 1, wherein the metal ion is a metal ion selected from a zinc ion, an iron ion, a cobalt ion, a nickel ion, a copper ion, or a silver ion.

3. The X-ray crystallographic sample-forming material according to claim 1 or 2, wherein the organic compound is an organic compound including a functional group capable of interacting with at least one group selected from a CONH group, a CO group, or an NH group.

4. The X-ray crystallographic sample-forming material according to claim 1 or 2, wherein the organic compound is an organic compound including at least one functional group selected from a substituted or unsubstituted amino group, a carboxyl group, an active methylene group, a nitrile group, a ketone group, an aldehyde group, an ester group, an ether group, an amide group, a hydroxy group, a halogen group, or a sulfonamide group.

5. The X-ray crystallographic sample-forming material according to claim 1 or 2, wherein the organic compound is an organic compound having a log P value of -5 or greater and 7 or less.

6. A method for determining a molecular structure of an organic compound (X), the method comprising steps 1 and 2:
step 1: allowing the organic compound (X) to infiltrate into the X-ray crystallographic sample-forming material described in claim 1 or 2, and fixing the organic compound (X) in cavities of the X-ray crystallographic sample-forming material; and
step 2: irradiating the X-ray crystallographic sample-forming material containing the fixed organic compound (X) with X-rays, analyzing obtained diffraction data, and determining a molecular structure of the organic compound (X).

7. A method for determining a molecular structure of a gaseous odor component (Y), the method comprising steps 1, 2, and 3:
step 1: feeding a sample gas containing the gaseous odor component (Y) to a column for separating gas components and fractionating the gaseous odor component (Y);
step 2: allowing the fractionated gaseous odor component (Y) to infiltrate into the X-ray crystallographic sample-forming material described in claim 1 or 2, and fixing the gaseous odor component (Y) in cavities of the X-ray crystallographic sample-forming material; and
step 3: irradiating the X-ray crystallographic sample-forming material containing the fixed gaseous odor component (Y) with X-rays, analyzing obtained diffraction data, and determining a molecular structure of the gaseous odor component (Y).
